(19) Europäisches Patentamt / European Patent Office / Office européen des brevets

(11) **EP 3 800 206 A1**

(12) **EUROPEAN PATENT APPLICATION**

(43) Date of publication:
07.04.2021 Bulletin 2021/14

(51) Int Cl.:
*C08B 37/00* (2006.01)        *C08K 5/00* (2006.01)
*C08L 5/00* (2006.01)

(21) Application number: **19201216.9**

(22) Date of filing: **02.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Clariant Produkte (Deutschland) GmbH 65929 Frankfurt am Main (DE)**

(72) Inventors:
• **LOEW, Sebastian**
  **82140 Olching (DE)**
• **LIST, Felix**
  **81476 München (DE)**
• **JIRAN, Sarah**
  **82256 Fürstenfeldbruck (DE)**

(74) Representative: **Graser, Konstanze Clariant Produkte (Deutschland) GmbH IPM / Patent & License Management Arabellastrasse 4a 81925 München (DE)**

(54) **BIO-BASED POLYSACCHARIDE FOIL**

(57)    The present invention is directed to a polysaccharide foil comprising a polysaccharide polymer and a plasticizer, wherein the polysaccharide polymer originates from fermentation of a bacterium or a fungus such as *Ascomycota.* The invention further relates to the use of the polysaccharide foil and a method for its production.

# Description

The present invention refers to a polysaccharide foil comprising a polysaccharide polymer originating from fermentation of a microorganism such as a bacterium or fungus, e.g., *Ascomycota* and a plasticizer, its use and a method for its production.

## Technical background

There is a continuously increasing demand of sustainable and so called "green" material in almost all technical fields. Thus, it is of high importance that new materials such as a foil, which is applicable in a wide variety of technical fields, is free of contaminants, is recyclable and based on a renewable resource, i.e., there is a high demand for environmentally friendly material. At the same time it is important that these materials provide high quality which is identical or even better than the quality of the common material, particularly if it is intended that the new material should replace the common material.

Since a long time, there is an unmet need in the field of foil production, suitable for example for packaging, which is based on plastic material to find environmentally friendly alternatives.

*Ascomycetes* are yeast-like fungi capable of producing a wide variety of different valuable bioproducts such as enzymes, pigments and biopolymers, e.g., polysaccharides such as beta-glucans. These beta-glucans are of high economic importance as they can be widely used in food, pharmaceutical, agricultural and also chemical applications.

However, a polysaccharide such as a beta-glucan was not considered to form a stable, in particular water-resistant, flexible foil. The present invention provides such foil comprising a polysaccharide polymer originating from the fermentation of a microorganism such as a bacterium or fungus, e.g., *Ascomycota* and a plasticizer.

## Summary

The present invention is directed to a polysaccharide foil comprising 0.1 - 10 wt.-% of a polysaccharide polymer and 0.1 - 10 wt.-%, 0.1 - 5 wt.-% or 0.1 - 1 wt.-% of a plasticizer, wherein the polysaccharide polymer originates from fermentation of *Ascomycota.* The polysaccharide polymer is for example beta-glucan.

The plasticizer of the polysaccharide foil is for example selected from the group of phthalate-based plasticizer, trimellitates, adipates, sebacates, maleates, azelates, terephthalates, benzoates, 1,2-cyclohexane dicarboxylic acid diisononyl ester, alkyl sulphonic acid phenyl ester, sulfonamides, organophosphates, glycols, polyethers, polymeric plasticizers, polybutene, bio-based plasticizer, plasticizers for energetic material or a combination thereof. The plasticizer is for example a polyalcohol, a sugar alcohol, a monosaccharide, such as glycerol and/or sorbitol, or a combination thereof,.

The polysaccharide foil of the present invention is for example water-resistant, permeable to vapor, in particular to water vapor, or a combination thereof. In addition, the polysaccharide foil retains alcohol such as ethanol. It is clear, transparent, flexible or a combination thereof. Moreover, the polysaccharide foil is thermostable for example up to 70 °C, up to 80 °C, up to 90 °C, up to 100 °C, up to 110 °C, up to 120 °C, up to 130 °C, up to 140 °C or up to 150 °C.

The polysaccharide foil comprises for example a colourant, a fragrance or a combination thereof. It is for example a packaging material, a composite, a textile, a medicinal product, a coating material, a cosmetic product, a food product, a varnish or part of a packaging material, of a composite, of a textile, of a medicinal product, of a coating material, of a cosmetic product, of a food product or of a varnish.

The present invention further relates to the use of the polysaccharide foil for the production of a packaging material, a composite, a textile, a medicinal product, a coating material, a cosmetic product, a food product, a varnish or a combination thereof. The medicinal product is for example selected from the group consisting of a wound closure, a bandage such as a waterproof bandage, a soluble film, a capsule resistant to gastric juice or a combination thereof.

Furthermore, the present invention refers to a method for the production of the polysaccharide foil, wherein the polysaccharide polymer originates from fermentation of *Ascomycota,* comprising the steps:

a) providing a medium with a dry matter content selected from the range of from 0.1 to 0.3 wt.-% and containing less than 0.1 wt.-% of an organic nitrogen source;
b) inoculating the medium with at least one fungus belonging to the division of *Ascomycota* with an inoculation density selected from the range of from 0.01 $g_{CDW}$/L to 50 $g_{CDW}$/L;
c) adding at least one carbon source in a concentration of from 1 to 20 wt.-%;
d) mixing the medium and the at least one fungus according to step d) for a time period of from 24 to 400 hours;
e) removing from 30 to 90 wt.-% of the medium and the at least one fungus;
f) adding from 30 to 90 wt.-% of a medium as defined in step a) additionally containing a carbon source;
g) repeating steps c) to f) from 2 to 100 times;
wherein at least one of steps c) to g) is carried out with a relative gas content selected from the range of from 0.005 to 0.5, and
h) adding a plasticizer.

The method for the production of a polysaccharide foil optionally further comprises the step of preparing an aqueous solution of the polysaccharide polymer in a concentration of 0.1 - 10 wt.-% or 0.5 - 5 wt.-%. The plasticizer is for example added in a concentration of 0.1 - 10 wt.-%, 0.1 - 5 wt.-% or 0.1 - 1 wt.-%.

Optionally, the method further comprises the step of depositing the polysaccharide foil on a hydrophobic surface such as a plastic surface which is for example sloping or flat, and drying the polysaccharide foil with an air stream such as a continuous air stream.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference. In particular, beta-glucans particularly suitable for the present invention are defined for example in EP 2018165787 which is herein incorporated by reference.

**Description of figures**

**Fig. 1** shows the preparation of a polysaccharide foil of the present invention.

**Fig. 2A and 2B** depict permeability for water vapor of the polysaccharide foil of the present invention in comparison to a parafilm.

**Detailed description**

The present invention is directed to a bio-based polysaccharide foil. It comprises or consists of a polysaccharide polymer and a plasticizer, wherein the polysaccharide polymer originates from fermentation of a microorganisms such as a bacterium or fungus, e.g., *Ascomycota*. The polysaccharide polymer is for example beta-glucan. Beta-glucan comprises a group of beta-D-glucose polysaccharides naturally occurring in the cell walls of cereals, bacteria, and fungi, respectively. At dietary intake levels of for example at least 3 g per day, beta-glucan decreases blood levels of LDL cholesterol and so may reduce the risk of cardiovascular diseases.

Thus, the polysaccharide foil of the present invention is highly economically friendly, has positive health aspects and is at the same time stable, water-resistant and flexible, i.e., ideal for example as a packaging material, composite material, textile material, medicinal product, coating material, cosmetic material, a food material or varnish material.

Throughout this description and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example", "specific", "specific embodiment"), provided herein is intended merely to better illustrate the invention and does not pose a limitation on the scope of the invention otherwise claimed. No language in the specification should be construed as indicating any non-claimed element essential to the practice of the invention.

The polysaccharide foil of the present invention is bio-based and 100 % recyclable. It comprises or consists of 0.1 - 10 wt.-% of a polysaccharide polymer and 0.1 - 10 wt.-%, 0.1 - 5 wt.-% or 0.1 - 1 wt.-% of a plasticizer, or it comprises or consists of 0.5 - 5 wt.-% of a polysaccharide polymer and 0.1 - 10 wt.-%, 0.1 - 5 wt.-% or 0.1 - 1 wt.-% of a plasticizer, or it comprises or consists of 0.3 - 3 wt.-% of a polysaccharide polymer and 0.1 - 10 wt.-%, 0.1 - 5 wt.-% or 0.1 - 1 wt.-% of a plasticizer, wherein the polysaccharide polymer originates from fermentation of a bacterium or fungus such as *Ascomycota.*

The bacterium and/or fungus produces numerous compounds amongst others a polysaccharide polymer such as beta-glucan. Beta-glucans are polysaccharides derived from glucose monomers. The monomers are linked by glycosidic bonds. Four types of glucose-based polysaccharides are possible: 1,6-bonded, 1,4-bonded, 1,3-bonded and 1,2-bonded glucans. Beta-glucans are for example cellulose (β-1,4-glucan), chrysolaminarin (β-1,3-glucan), curdlan (β-1,3-glucan), laminarin (β-1,3- and β-1,6-glucan), lentinan (β-1,6:β-1,3-glucan), lichenin (β-1,3- and β-1,4-glucan), oat beta-glucan (β-1,3- and β-1,4-glucan), pleuran (β-1,3- and β-1,6-glucan) or zymosan (β-1,3-glucan). The polysaccharide polymer of the polysaccharide foil of the present invention is for example beta-glucan.

The polysaccharide foil of the present invention is water resistant, resistant to alcohol such as methanol or ethanol and/or permeable to vapor such as water vapor. Thus, the polysaccharide foil allows for example separating water and alcohol. Further, the polysaccharide foil is clear, transparent and/or flexible. It is not brittle which is for example supported by the plasticizer.

The plasticizer of the polysaccharide foil of the present invention is for example selected from the group of phthalate-based plasticizer, trimellitates, adipates, sebacates, maleates, azelates, terephthalates, benzoates, 1,2-cyclohexane dicarboxylic acid diisononyl ester, alkyl sulphonic acid phenyl ester, sulfonamides, organophosphates, glycols, polyethers, polymeric plasticizers, polybutene, bio-based plasticizer, plasticizers for energetic material or a combination thereof. The plasticizer is for example a polyalcohol, a sugar alcohol, a monosaccharide, such as glycerol and/or sorbitol, or a combination thereof.

Moreover, the polysaccharide foil of the present invention is thermostable for example in the range of 10 °C to 95 °C, 15 °C to 90 °C, 20 °C to 85 °C, 25 °C to 80 °C, 30 °C to 75 °C, 40 °C to 70 °C, 50 °C to 60 °C, or up to 40 °C, up to 50 °C, up to 60 °C, up to 70 °C, up to 80 °C, up to 90 °C or up to 95 °C.

The polysaccharide foil of the present invention optionally comprises an additive such as a colourant and/or a fragrance. The polysaccharide foil comprising fragrance is for example used in fragrance encapsulation in cleaning agent such as washing agent.

Further, the polysaccharide foil of the present invention is for example a packaging material for example in general in all types of industry or more specific in food industry, where the food product is for example wrapped in the polysaccharide foil or the polysaccharide foil is directly administered on the food product, e.g., sprayed on fruits. Alternatively, the polysaccharide foil is for example applied to a packaging on the basis of paper or carton, where it forms aprotective layer such as a film against water or a hydrophobic substance such as mineral oil saturated hydrocarbons (MOSH) and/or mineral oil aromatic hydrocarbons (MOAH). The polysaccharide foil may form the basis for a candy with or without aromatic compounds. This may even result in health advantages, if the polysaccharide is beta-glucan which positively effects the cholesterol level in human or animal bodies.

Alternatively, the polysaccharide foil is a composite for example in a Tetra Pak or a textile or forms part in a composite or a textile such as Goretex which is water resistant and permeable for vapor such as water vapor.

Alternatively, the polysaccharide foil is a medicinal product such as a wound closure, a bandage such as a waterproof bandage, a soluble film, and/or a capsule resistant to a body fluid such as gastric juice. The polysaccharide foil may form a sprayable plaster.

Alternatively, the polysaccharide foil is a coating or part of a coating, e.g., for a membrane for example for laboratory use or for a plastic for example in the automotive industry.

Alternatively, the polysaccharide foil of the present invention is a cosmetic product or part of a cosmetic product, e.g., a face mask, a crème such as sun crème for UV protection or an epilating product.

Moreover, the polysaccharide is a varnish or part of a varnish, e.g., a water-based varnish including nail varnish.

Thus, the polysaccharide polymer of the present invention can be used for the production of all of these products such as a packaging material, a composite, a textile, a medicinal product, a coating material, a cosmetic product, a food product and/or a varnish.

The polysaccharide foil is recyclable for example by breaking-up the foil such as grinding the foil and rehydrating the foil, e.g., in water. Alternatively, the polysaccharide foil is rehydrated in a basic (e.g., pH >13) or acidic (e.g., pH < 2) solution, by growing a microorganism such as a bacterium or fungus, e.g., Ascomycota, on the polymer foil or by use of an enzyme such as beta-glucosidase.

The polysaccharide polymer forming part of the polysaccharide foil of the present invention originates from fermentation of a bacterium or fungus such as *Ascomycota.* Therefore, the present invention refers for example to a method for the production of a polysaccharide foil, wherein the polysaccharide polymer originates from fermentation of *Ascomycota,* comprising the steps:

a) providing a medium with a dry matter content selected from the range of from 0.1 to 0.3 wt.-% and containing less than 0.1 wt.-% of an organic nitrogen source;
b) inoculating the medium with at least one fungus belonging to the division of *Ascomycota* with an inoculation density selected from the range of from 0.01 $g_{CDW}$/L to 50 $g_{CDW}$/L;
c) adding at least one carbon source in a concentration of from 1 to 20 wt.-%;
d) mixing the medium and the at least one fungus according to step d) for a time period of from 24 to 400 hours;
e) removing from 30 to 90 wt.-% of the medium and the at least one fungus;
f) adding from 30 to 90 wt.-% of a medium as defined in step a) additionally containing a carbon source;
g) repeating steps c) to f) from 2 to 100 times; wherein at least one of steps c) to g) is carried out with a relative gas content selected from the range of from 0.005 to 0.5;
h) optionally filtrating the medium comprising the fungus to remove cell mass;
i) optionally one or more washing steps; and
j) adding a plasticizer.

The method optionally further comprises a precipitation step, wherein the polysaccharide polymer is precipitated from the medium after the filtration step h) or from the washing solution after one or more washing steps i). The precipitation is for example performed using an alcohol such as ethanol. The precipitated polysaccharide polymer can be rehydrated which allows for example exact dosing of the polysaccharide polymer in solution.

The method optionally further comprises the step of depositing the polysaccharide foil on a hydrophobic surface such as a plastic surface which is for example sloping or flat. The polysaccharide foil is for example dried with an air stream such as a continuous air stream. The temperature of the air stream is for example 15 - 60 °C, 20 - 55 °C, 25 °C - 50 °C, 30 °C - 45 °C or 40 °C. The polysaccharide foil is for example dried with the air stream such as a continuous air stream for 1 - 24 h, 5 to 20 h, 10 to 15 h, or 1, 2 or 3 days. The air stream is 330 to 650 $m^3/h$, 400 to 600 $m^3/h$, 450 to 550 $m^3/h$ or 400 to 500 $m^3/h$, or 500 $m^3/h$.

Within the present invention the term "*Ascomycota*" is to be understood to comprise any fungus belonging to the division or phylum of the kingdom Fungi. *Ascomycota* belong to the subkingdom *Dikarya*. *Ascomycota* which are suitable for the method of the present invention include but are not limited to any fungus falling in the class of *Dothideomycetes* such as *Aureobasidiaceae*. The method for the production of the polysaccharide foil is for example based on the fermentation of *Aureobasidium pullulans* also referred to as *Aureobasidium oleae, Azymocandida malicola, Candida malicola, Cladosporium pullulans, Dematium pullulans, Exobasidium vitis, Hormonema oleae, Hormonema pullulans, Pullularia fermentans, Pullularia fermentans var. schoenii, Pullularia pullulans, Torula oleae* or *Torula schoenii*. All of which are to be understood as synonyms. Fungi belonging to the division of *Ascomycota* are commonly referred to as *Ascomycetes.*

If the fungus for example belonging to the group of *Ascomycota* grows in the presence of UV light or is even irradiated with UV light, it produces melanin. Melanin is a broad term for a group of natural pigments found in most organisms. The chemical formula of melanin is for example $C_{18}H_{10}N_2O_4$. Melanogenesis is initiated by exposure to UV radiation and melanin is an effective absorbent of light. The pigment is able to dissipate over 99.9% of absorbed UV radiation. The three basic types of melanin are eumelanin, pheomelanin, and neuromelanin. The most common type is eumelanin, of which there are two types-brown eumelanin and dark brown eumelanin. Thus, in the presence of UV light a fungus for example belonging to *Ascomycota* produce a dark, e.g., brown or black polysaccharide such as beta-glucan.

The polysaccharide foil of the present invention contains for example from 0.05 to 30 g/l melanin, wherein a melanin content of from 0.05 to 15 g/l, from 0.07 to 7 g/l or from 0.1 to 6 g/l is also suitable.

The medium to be provided within step a) of the method for the production of a polysaccharide foil is characterized by a dry matter content selected from the range of from 0.1 to 0.3 wt.-%, wherein a dry matter content selected from the range of from 0.15 to 0.25 wt.-% is also within the scope of the present invention. The term "dry matter content" refers to the mass determined after water and other volatile compounds have been removed from the sample using an IR-balance.

At least one of steps c) to g) of the method for the production of a polysaccharide foil of the present invention is carried out at a relative gas content selected from the range of from 0.005 to 0.50, wherein ranges of from 0.0075 to 0.40, from 0.01 to 0.35 and from 0.01 to 0.20 are also within the scope of the present invention.

Within the present invention the relative gas content is defined by the formula:

$$rG = \left(\frac{P}{V_L * \rho_L}\right)^{0,28} * u_G^{0,7}$$

(wherein P is the power input [W], $V_L$ is the volume of the medium, the at least one fungus and the at least one carbon source [$m^3$], $\rho_L$ is the density of the medium according to step a), the at least one fungus and the at least one carbon source [$kg/m^3$], and $u_G$ is the superficial gas velocity [m/s] (Liepe (1988): Verfahrenstechnische Berechnungsmethoden Teil 4: Stoffvereinigen in fluiden Phasen - Ausrüstungen und ihre Berechnungen von einem Autorenkollektiv unter Federführung von F. Liepe, Leipzig, VEB Deutscher Verlag für Grundstoffindustrie).

Within the present invention the term "power input" is defined as $P = N_e * \rho_L * n^3 * d^5$ (wherein Ne is the Newton number, $\rho_L$ is the density of the medium according to step a), the at least one fungus and the at least one carbon source [$kg/m^3$], n is the frequency of the stirrer [$s^{-1}$], d is the diameter of the stirrer [m]). Within the present invention the term superficial gas velocity is defined as

$$u_g = \frac{4 * Q_G}{Dr^2 * \pi},$$

wherein $Q_G$ is the volumetric gas flow [$m^3/s$], to be understood as the volume of air sparged into the medium, the at least on fungus and the at least one carbon source per second, and Dr is the inner diameter of the vessel.

The medium may be provided in any vessel known to a person skilled in the art as suitable for the method for the production of a polysaccharide foil, such as a batch or fed-batch reactor, an air lift reactor, a stirred tank reactor or a bubble column reactor. The absolute pressure in the headspace of the vessel is for example selected from the range of from 1 to 11 bar, wherein ranges of from 1.1 to 10 bar, from 1.2 to 9 bar and from 1.3 to 8 bar are also within the scope of the present invention. The vessel is for example equipped with a light source providing a light intensity of at least 300 Lux. The light source is for example placed above the surface of the fermentation medium during at least 40 % of the time as described in step d). 1 Lux is defined as 1 Lumen per $m^2$. Alternatively or in addition, the light source is for example placed next to and outside of the vessel. In this case the vessel is for example equipped with a window or may comprise or consist of transparent material. Alternatively or in addition, the light source is for example placed inside the vessel submerged in the fermentation medium.

Within step b) of the method for the production of a polysaccharide foil the medium is inoculated with at least one fungus belonging to the division of *Ascomycota* with an inoculation density selected from the range of from 0.01 $g_{CDW}$/L to 50 $g_{CDW}$/L. The "inoculating" may be carried out by any method known to a person skilled in the art as suitable for the method of the present invention such as addition of the at least one fungus in a purified form or in form of a pre-culture. Within the method of the present invention, the concentration of the at least one fungus is selected from a range of from 0.01 $g_{CDW}$/L to 50 $g_{CDW}$/L, wherein ranges of from 0.02 $g_{CDW}$/L to 25 $g_{CDW}$/L, of from 0.03 $g_{CDW}$/L to 20 $g_{CDW}$/L and of from 0.05 $g_{CDW}$/L to 15 $g_{CDW}$/L are also within the scope of the invention. The parameter "L" thereby relates to the volume of the medium according to step a) of the method of the present invention.

The at least one fungus used in the method for the production of a polysaccharide foil is for example only one single type of fungus or a mixture of different fungi. The term "cell dry weight" (CDW) is to be understood as the weight of the total biomass divided by the volume of a fermentation sample after centrifugation of a fermentation sample, removal of the supernatant, washing with a solution of 9 g/L NaCl, again centrifugation, again removal of the supernatant and drying at 90 °C until constant weight is reached.

The term "pre-culture" according to the present invention is to be understood to comprise any composition of fermentation or culture medium used for pre-cultivating the at least one fungus. The term "pre-culture" is well known to any person skilled in the art of fermenting. The at least one fungus is pre-cultivated for example up to a cell dry weight of 2 $g_{CDW}$/L - 30 $g_{CDW}$/L before addition to the medium according to step b) of the method of the present invention. It is a particular advantage of this method that high yields of beta glucan can be obtained while keeping the volume of pre-culture relatively low, as for example the use of from 0.1 to 0.5 wt.-% (weight pre-culture to weight medium) is sufficient.

The nitrogen content of the medium according to step a) is for example selected from the range of from 0.003 to 0.02 wt.-%, wherein a range of from 0.005 to 0.015 wt.-% and from 0.006 to 0.01 wt.-% is also within the scope of the present invention.

Step b) of the method of the present invention is for example carried out for a time period of from 1 minutes to 8 hours, wherein a time period of from 2 minutes to 7 hours, from 3 minutes to 6 hours and form 5 minutes to 5 hours is also within the scope of the present invention. It is thereby particularly suitable to carry out inoculation by adding the at least one fungus all at once at the beginning of the time period.

Within step c) of the method for the production of a polysaccharide foil, at least one carbon source is for example added in a concentration of from 1 to 20 wt.-%, wherein a concentration of from 2 to 18 wt.-%, from 3 to 15 wt.-% and from 4 to 12 wt.-% is also within the scope of the present invention. The "adding" may be carried out by any method known to a person skilled in the art as suitable for the method of the present invention. Step c) is for example carried out after step b) of the method of the present invention but may also be carried out before step b) or steps b) and c) may also be carried out concurrently. Alternatively, steps a) and c) may be carried out concurrently and before step b).

Within step d) of the method of the present invention the medium and the at least one fungus are mixed for a time period of from 24 to 400 hours, wherein a time period of from 30 to 350 hours, from 35 to 300 hours, from 40 to 250 hours and from 50 to 200 hours are also within the scope of the present invention. The "mixing" may be carried out by any method known to a person skilled in the art as suitable for the method of the present invention.

A method which has shown to be of specific advantage regarding time and energy consumption is mixing by continuously pumping of the medium and at least one fungus from the bottom to the top of the vessel.

The mixing is for example carried out without the use of any kind of rotating device such as a stirrer. A system which is advantageous for carrying out the method of the present invention is for example a bubble column reactor.

The mixing is for examle carried out with a specific power input P/V of from 2 to 12.000 W/m³, wherein a specific power input of from 100 to 1.000 W/m³ is also within the scope of the present invention.

Within a stirred mixing system such as a stirred tank reactor, the term "specific power input" is to be understood as

$$\frac{P}{V} = \frac{Ne * \rho_L * n^3 * d^5}{V},$$

wherein P is the power input [W], Ne is the Newton number [-], $\rho$ is the density of the medium according to step a) [kg/m³], n is the stirrer frequency [s$^{-1}$], d is the diameter of the impeller [m] and V is the volume of the medium according to step a), the carbon source and the at least one fungus. Within a system without any kind of impellor or stirrer such as a bubble column reactor, the specific power input is to be understood as

$$\frac{P}{V} = \rho * g * u_g,$$

wherein g is the gravitational acceleration [m s$^{-2}$], and $u_g$ is the superficial gas velocity as defined above.

The mixing is for example carried out until the dynamic viscosity at a shear rate of 20 s$^{-1}$ and at a temperature of 20 °C of the medium and at least one fungus during step d) is within the range of from 40 to 600 mPas, wherein a range of from 50 to 550 mPas, from 70 to 500 mPas, from 90 to 450 mPas and from 100 to 400 mPas is also within the scope of the present invention.

The pH is for example kept in the range of from 4.0 to 6.0 during the whole process, wherein a range of from 4.25 to 5.75, from 4.25 to 5.5 and from 4.25 to 4.75 is also within the scope of the present invention. Alternatively, the pH is for example kept above 4.75, such as within a range of from 4.75 and 5.25 during the complete process by addition of a base, wherein bases can be any bases known to person trained in the skill of fermentation to be suitable for the use in fermentations and not containing nitrogen, such as NaOH or KOH. It is of particular advantage of the method of the present invention that no acid needs to be used to adjust the pH levels.

The temperature is for example kept in the range of from 22 to 30 °C during the whole production method, wherein a range of from 23 to 30 °C, a range from 24 to 29 and from 24 to 28 °C is also within the scope of the present invention.

Within step e) of the method for the production of a polysaccharide foil, from 30 to 90 wt.-% of the medium and at least one fungus are removed, wherein ranges of from 40 to 85 wt.-%, from 45 to 80 wt.-%, from 50 to 75 wt.-% and from 55 to 65 wt.-% are also within the scope of the present invention. The "removing" can thereby be carried out by any means and method known to a person skilled in the art as suitable for the inventive process.

Within step f) of the method of the present invention, from 30 to 90 wt.-% of a medium as defined within step a) of the inventive process and additionally containing a carbon source are added to the remaining medium and at least one fungus, wherein ranges of from 40 to 85 wt.-%, from 45 to 80 wt.-%, from 50 to 75 wt.-% and from 55 to 65 wt.-% are also within the scope of the present invention. It is thereby understood that the range of from 30 to 90 wt.-% comprises the weight of the medium and the carbon source. The "adding" can thereby be carried out by any means and method known to a person skilled in the art as suitable for the inventive process. It is thereby of importance that the amount of removed medium and fungus and the amount of new medium are equal, wherein a deviation of up to 30%, preferably less than 10 % is tolerable.

Within step g) of the method of the present invention steps c) to f) are repeated from 2 to 100 times, wherein a repetition of from 2 to 80, from 2 to 70, from 2 to 50 and from 2 to 25 times is also within the scope of the present invention. It is a particular advantage of the method of the present invention that cells and fermentation products can be harvested up to 100 times without the need of new inoculation. The fermentation process and medium composition of the method of the present invention guarantees high yields of the polysaccharide polymer such as beta glucan. The repetition of steps c) to f) is of particular advantage as the yield of polysaccharide polymer such as beta-glucan increases significantly with the first repetition. In addition, cleaning expenditures can be minimized as cleaning of the reactor is only necessary after the completion of step g) of the method of the present invention contributing to further cost savings, costs for the preparation of precultures can be minimized as preculture preparation is only necessary for every second to 100th iteration of the process. Additionally downtimes (non-productive phases) of the production equipment are minimized, since the production can be kept running without time losses for harvesting, sterilization, cleaning and filling. It is, however, also possible to obtain decent yields of beta glucan by not repeating steps c) to f) but carrying out steps c) to f) just once.

Within the present invention the term "polysaccharide polymer" is to be understood as any polysaccharide polymer produced by living organisms. The polysaccharide polymer shows for example a temperature-independent viscosity within the range from 20 to 100°C or within the range of from 20 to 80°C. The polysaccharide polymer contains for example monomeric units which are covalently bonded such as polymeric carbohydrate structures, rubber, suberin, melanin and lignin. The polysaccharide polymer has for example a shear thinning behavior described by a constant $b$ in which $b$ is the gradient between two pairs of values $x1/y1$ and $x2/y2$ where $x$ is the shear rate [s$^{-1}$] in the range of 0.1-100 s$^{-1}$ and $y$ is the dynamic viscosity [mPas] at the given shear rate and at a temperature of the biopolymer between 20°C and 80°C and a concentration between 0.05 and 0.5 wt.-%. The constant $b$ is for example described by the formula $b = -((lg(y1/y2)/(lg(x1/x2))$. $b$ is for example selected from the range of from 0.65 to 1.05. Alternatively, $b$ is for example selected from the range of from 0.7 to 1.0 or for example from the range of from 0.75 to 0.9. Within the present invention the term "beta-glucan" is to be understood as referring to any β-D-glucose polysaccharide characterized by the formation of a linear backbone with 1-3 β-glycosidic bonds. The beta-glucans have for example a molecular weight distribution with a maximum of from 0.5 to 5.0 Million Dalton (MegaDalton) or from 0.75 to 3.5 Million Dalton (MegaDalton) or from 1.0 to 2.0 Million Dalton (MegaDalton).

In the following, the elements of the fermentation of *Ascomycota* will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

## Specific embodiments

The following specific embodiments define embodiments which are particularly advantageous for the production of a polysaccharide polymer such as beta-glucan by a fungus belonging to the family of *Aureobasidiaceae*. These embodiments are not meant to limit the scope of the present invention in any respect.

Specific embodiment A

Process for the fermentation of *Aureobasidiaceae* comprising the following steps:

    a) Providing a medium with a dry matter content selected from the range of from 0.1 to 0.3 wt.-% and containing less than 0.1 wt.-% of an organic nitrogen source;

    b) Inoculating the medium with at least one fungus belonging to the species of *Aureobasidium pullulans* with an inoculation density selected from the range of from 0.01 $g_{CDW}$/L to 50 $g_{CDW}$/L;

    c) Adding at least one carbon source in a concentration of from 1 to 20 wt.-%;

    d) Mixing the medium and the at least one fungus according to step d) for a time period of from 24 to 400 hours;

    e) Removing from 30 to 90 wt.-% of the medium and the at least one fungus;

    f) Adding from 30 to 90 wt.-% of a medium as defined in step a) additionally containing a carbon source;

    g) Repeating steps c) to f) from 2 to 100 times; wherein at least one of steps c) to g) is carried out with a relative gas content selected from the range of from 0.005 to 0.5;

    h) optionally filtrating the medium comprising the fungus to remove cell mass;

    i) optionally one or more washing steps and

    j) Adding a plasticizer, e.g., in a concentration of 0.1 - 10 wt.-%, 0.1 - 5 wt.-% or 0.1 - 1 wt.-%.

Specific embodiment B

Process for the fermentation of *Aureobasidiaceae* comprising the following steps:

    a) Providing a medium with a dry matter content selected from the range of from 0.1 to 0.3 wt.-% and containing less than 0.1 wt.-% of an organic nitrogen source;

    b) Inoculating the medium with at least one fungus belonging to the species of *Aureobasidium pullulans* with an inoculation density selected from the range of from 0.01 $g_{CDW}$/L to 50 $g_{CDW}$/L;

    c) Adding sucrose in a concentration of from 1 to 20 wt.-%;

    d) Mixing the medium and the at least one fungus according to step d) for a time period of from 24 to 400 hours;

    e) Removing from 30 to 90 wt.-% of the medium and the at least one fungus;

    f) Adding from 30 to 90 wt.-% of a medium as defined in step a) additionally containing a carbon source;

    g) Repeating steps c) to f) from 2 to 100 times; wherein at least one of steps c) to g) is carried out with a relative gas content selected from the range of from 0.005 to 0.2,

    h) optionally filtrating the medium comprising the fungus to remove cell mass;

    i) optionally one or more washing steps and

    j) Adding a plasticizer, e.g., in a concentration of 0.1 - 10 wt.-%, 0.1 - 5 wt.-% or 0.1 - 1 wt.-%.

Specific embodiment C

Process for the fermentation of *Aureobasidiaceae* comprising the following steps:

    a) Providing a medium with a dry matter content selected from the range of from 0.1 to 0.3 wt.-% and containing less than 0.1 wt.-% of an organic nitrogen source;

    b) Inoculating the medium with at least one fungus belonging to the species of *Aureobasidium pullulans* with an inoculation density selected from the range of from 0.01 $g_{CDW}$/L to 50 $g_{CDW}$/L;

    c) Adding sucrose in a concentration of from 1 to 20 wt.-%;

    d) Mixing the medium and the at least one fungus according to step d) for a time period of from 24 to 400 hours;

    e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times; wherein at least one of steps c) to g) is carried out with a relative gas content selected from the range of from 0.005 to 0.2 and at a temperature of from 24 to 30 °C and at a pH of from 4 to 6,

h) optionally filtrating the medium comprising the fungus to remove cell mass;

i) optionally one or more washing steps and

j) Adding a plasticizer, e.g., in a concentration of 0.1 - 10 wt.-%, 0.1 - 5 wt.-% or 0.1 - 1 wt.-%.

Specific embodiment D

Process for the fermentation of *Aureobasidiaceae* comprising the following steps:

a) Providing a medium with a dry matter content selected from the range of from 0.1 to 0.3 wt.-% and containing less than 0.1 wt.-% of an organic nitrogen source;

b) Inoculating the medium with at least one fungus belonging to the species of *Aureobasidium pullulans* with an inoculation density selected from the range of from 0.01 $g_{CDW}$/L to 50 $g_{CDW}$/L;

c) Adding sucrose in a concentration of from 1 to 20 wt.-%;

d) Mixing the medium and the at least one fungus according to step d) for a time period of from 24 to 400 hours;

e) Removing from 30 to 90 wt.-% of the medium and the at least one fungus;

f) Adding from 30 to 90 wt.-% of a medium as defined in step a) additionally containing a carbon source;

g) Repeating steps c) to f) from 2 to 100 times; wherein at least one of steps c) to g) is carried out with a relative gas content selected from the range of from 0.005 to 0.2 and at a temperature of from 24 to 30 °C and at a pH of from 4 to 6 and step b) is carried out for a time period of from 1 minute to 8 hours before step c),

h) optionally filtrating the medium comprising the fungus to remove cell mass;

i) optionally one or more washing steps and

j) Adding a plasticizer, e.g., in a concentration of 0.1 - 10 wt.-%, 0.1 - 5 wt.-% or 0.1 - 1 wt.-%.

The methods of all these embodiments optionally further comprise a precipitation step, wherein the polysaccharide polymer is precipitated from the medium after the filtration step h) or from the washing solution after one or more washing steps i). The precipitation is for example performed using an alcohol such as ethanol. The precipitated polysaccharide polymer can be rehydrated which allows for example exact dosing of the polysaccharide polymer in solution.
All these embodiments result in a polysaccharide foil of the present invention.

**Examples**

The following examples illustrate the present invention in more detail, however, the invention is not limited to these examples.

**Example 1:** Preparation of a polysaccharide foil

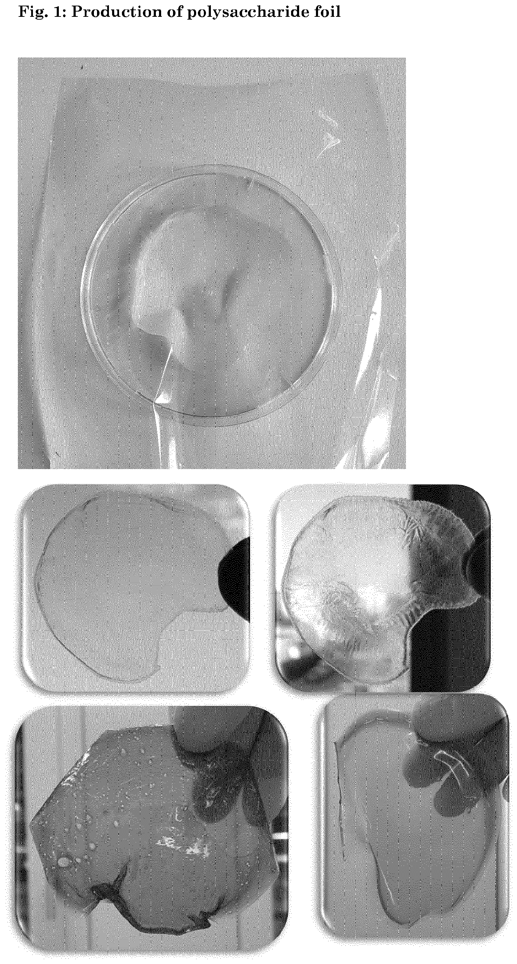

10 ml polysaccharide foil (5 g/l polysaccharide polymer content) was poured on a plastic foil over a Petri dish to create a cavity (Fig. 1A). The polysaccharide foil was allowed drying for 3 days and was separated from the plastic foil thereafter (Fig. 1B to 1E). Preparation of a polysaccharide foil of the present invention is shown in Fig. 1A to 1E.

**Example 2:** Preparation of a polysaccharide foil

To 100 ml polysaccharide medium (6 g/l polysaccharide polymer content) was added 1 g glycerol and the mixture poured on a plastic foil. The polysaccharide foil was dried for 10 hours in a constant airstream of 500 m$^3$/h and was separated from the plastic foil thereafter.

**Example 3:** Preparation of a polysaccharide foil

To 50 ml polysaccharide medium (4 g/l polysaccharide polymer content) was added 1 g sorbitol and the mixture poured on a plastic plate. The polysaccharide foil was dried for 5 hours in a constant airstream of 500 m$^3$/h and was separated from the plastic plate thereafter.

**Example 4:** Preparation of a polysaccharide foil

To 70 ml polysaccharide medium (10 g/l polysaccharide polymer content) was added 0.5 g menthol and the mixture poured on a plastic foil. The polysaccharide foil was dried for 8 hours in a constant airstream of 500 m$^3$/h and was separated from the plastic plate thereafter. The foil exhibited a menthol odor.

**Example 5:** Preparation of a polysaccharide foil

50 ml polysaccharide medium (5 g/l polysaccharide polymer content) were added to 200 ml ethanol. The solid was removed by filtration and subsequently rehydrated in 50 ml demineralized water. This solution was poured on a plastic surface and dried for 1 hour in a constant airstream of 500 m$^3$/h.

**Example 6:** Preparation of a polysaccharide foil

To 20 ml polysaccharide medium (5 g/l polysaccharide polymer content) was added 100 µl blue food coloring and the mixture was poured on a ceramic surface. The polysaccharide foil was dried for 10 hours in a constant airstream of 500 m$^3$/h.

**Example 7:** Test for thermostability of the polysaccharide foil

A polysaccharide foil was prepared according to the method of the present invention and tested for thermostability. The foil was heated on a plate to 70 °C for 2 h. The experiment shows that the polysaccharide foil was unchanged, i.e., is thermostabil at a temperature of 70 °C.

**Example 8:** Test for water permeability of the polysaccharide foil

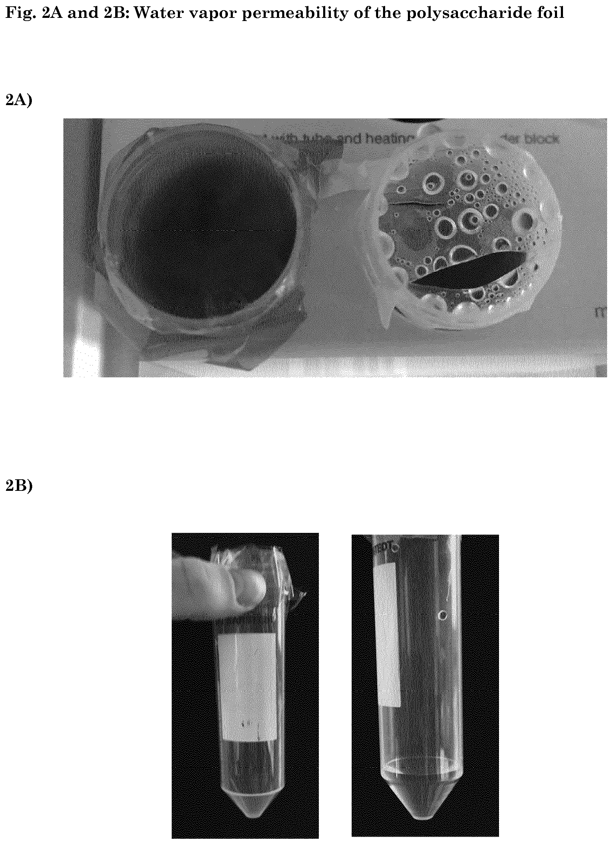

In these experiments two tubes (falcon tubes) were filled with 10 ml of water. One tube was closed with a polymer of the present invention, the other tube was closed with a parafilm. The tube covered with the polysaccharide foil which is unchanged after an incubation of the tube at 70 °C for 16 h does not comprise water anymore as water vapor evaporated through the polysaccharide foil. In the tube covered with the parafilm, the water vapor condenses at the parafilm which is not permeable for the water vapor (Fig. 2A and 2B).

**Example 9:** Water resistance test

A polysaccharide foil was prepared according to the method of the present invention and tested for water resistance in comparison to a parafilm and a polysaccharide foil prepared according to the method of the present invention containing 1 % w/w sorbitol and 0.1 % w/w food color. To each foil a drop of water was added and incubated for 10 h. The visual analysis showed no sign of structural degradation of the foil of the present invention.

**Claims**

1. Polysaccharide foil comprising 0.1 - 10 wt.-% of a polysaccharide polymer and 0.1 - 10 wt.-%, 0.1 - 5 wt.-% or 0.1 - 1 wt.-% of a plasticizer, wherein the polysaccharide polymer originates from fermentation of *Ascomycota*.

2. Polysaccharide foil according to claim 1, wherein the polysaccharide polymer is beta-glucan.

3. Polysaccharide foil according to claim 1 or 2, wherein the plasticizer is selected from the group of phthalate-based plasticizer, trimellitates, adipates, sebacates, maleates, azelates, terephthalates, benzoates, 1,2-cyclohexane dicarboxylic acid diisononyl ester, alkyl sulphonic acid phenyl ester, sulfonamides, organophosphates, glycols, polyethers, polymeric plasticizers, polybutene, bio-based plasticizer, plasticizers for energetic material or a combination thereof.

4. Polysaccharide foil according to any one claims 1 to 3, wherein the polysaccharide foil is water-resistant, permeable to vapor, in particular to water vapor, or a combination thereof.

5. Polysaccharide foil according to any one claims 1 to 4, wherein the polysaccharide foil retains alcohol such as ethanol.

6. Polysaccharide foil according to any one claims 1 to 5, wherein the polysaccharide foil is clear, transparent, flexible or a combination thereof.

7. Polysaccharide foil according to any one claims 1 to 5, wherein the polysaccharide foil is thermostable for example up to 70 °C, up to 80 °C, up to 90 °C, up to 100 °C, up to 110 °C, up to 120 °C, up to 130 °C, up to 140 °C or up to 150 °C.

8. Polysaccharide foil according to any one claims 1 to 7, wherein the polysaccharide foil comprises a colourant, a fragrance or a combination thereof.

9. Polysaccharide foil according to any one claims 1 to 8, wherein the polysaccharide foil is a packaging material, a composite, a textile, a medicinal product, a coating material, a cosmetic product, a food product, a varnish or part of a packaging material, of a composite, of a textile, of a medicinal product, of a coating material, of a cosmetic product, of a food product or of a varnish.

10. Use of a polysaccharide foil according to any one of claims 1 to 9 for the production of a packaging material, a composite, a textile, a medicinal product, a coating material, a cosmetic product, a food product, a varnish or a combination thereof.

11. Use of a polysaccharide foil according to claim 10, wherein the medicinal product is selected from the group consisting of a wound closure, a bandage such as a waterproof bandage, a soluble film, a capsule resistant to gastric juice or a combination thereof.

12. Method for the production of a polysaccharide foil according to any one of claims 1 to 9, wherein the polysaccharide polymer originates from fermentation of *Ascomycota,* comprising the steps:

> a) providing a medium with a dry matter content selected from the range of from 0.1 to 0.3 wt.-% and containing less than 0.1 wt.-% of an organic nitrogen source;
> b) inoculating the medium with at least one fungus belonging to the division of *Ascomycota* with an inoculation density selected from the range of from 0.01 $g_{CDW}$/L to 50 $g_{CDW}$/L;
> c) adding at least one carbon source in a concentration of from 1 to 20 wt.-%;
> d) mixing the medium and the at least one fungus according to step d) for a time period of from 24 to 400 hours;
> e) removing from 30 to 90 wt.-% of the medium and the at least one fungus;f) adding from 30 to 90 wt.-% of a medium as defined in step a) additionally containing a carbon source;
> g) repeating steps c) to f) from 2 to 100 times; wherein at least one of steps c) to g) is carried out with a relative gas content selected from the range of from 0.005 to 0.5, and
> h) adding a plasticizer.

13. Method for the production of a polysaccharide foil according to claim 12, further comprising the step of preparing an aqueous solution of the polysaccharide polymer in a concentration of 0.1 - 10 wt.-% or 0.5 - 5 wt.-%.

14. Method for the production of a polysaccharide foil according to claim 12 or 13, wherein the plasticizer is added in a concentration of 0.1 - 1 wt.-%.

15. Method for the production of a polysaccharide foil according to any one of claims 12 to 14 further comprising the step of depositing the polysaccharide foil on a hydrophobic surface and drying the polysaccharide foil with an air stream.

Fig. 1: Production of polysaccharide foil

Fig. 2A and 2B: Water vapor permeability of the polysaccharide foil

2A)

2B)

Europäisches
Patentamt

European
Patent Office

Office européen
des brevets

# EUROPEAN SEARCH REPORT

Application Number

EP 19 20 1216

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LAZARIDOU A ET AL: "Molecular weight effects on solution rheology of pullulan and mechanical properties of its films", CARBOHYDRATE POLYMERS, APPLIED SCIENCE PUBLISHERS, LTD. BARKING, GB, vol. 52, no. 2, 1 May 2003 (2003-05-01), pages 151-166, XP004405543, ISSN: 0144-8617, DOI: 10.1016/S0144-8617(02)00302-8 * paragraphs [0001], [02.1]; figure 5; tables 3,4 * | 1,3-11 | INV. C08B37/00 C08K5/00 C08L5/00 |
| X | NILAY KANDEMIR ET AL: "Production of Antimicrobial Films by Incorporation of Partially Purified Lysozyme into Biodegradable Films of Crude Exopolysaccharides Obtained from Aureobasidium pullulans Fermentation", FOOD TECHNOLOGY AND BIOTECHNOLOGY, vol. 43, no. 4, 1 January 2005 (2005-01-01), pages 343-350, XP055157357, * page 345; figures 2,3 * | 1,3-15 | |
| A | Y Göksungur ET AL: "Production of Pullulan From Beet Molasses ans Synthetic Medium by Aureobasidium pullulans", Turk J Biol Vol 28, 1 January 2004 (2004-01-01), pages 23-30, XP055678690, Retrieved from the Internet: URL:http://journals.tubitak.gov.tr/biology/issues/biy-04-28-1/biy-28-1-4-0311-3.pdf [retrieved on 2020-03-23] * page 24 * | 1-15 | TECHNICAL FIELDS SEARCHED (IPC) C08B C08K C08L |
| X | EP 0 236 124 A2 (HAYASHIBARA BIOCHEM LAB [JP]) 9 September 1987 (1987-09-09) * page 4; examples * | 1-11 | |

The present search report has been drawn up for all claims

| Place of search | Date of completion of the search | Examiner |
|---|---|---|
| Munich | 24 March 2020 | Vaccaro, Eleonora |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 20 1216

This annex lists the patent family members relating to the patent documents cited in the above-mentioned European search report.
The members are as contained in the European Patent Office EDP file on
The European Patent Office is in no way liable for these particulars which are merely given for the purpose of information.

24-03-2020

| Patent document cited in search report | | Publication date | Patent family member(s) | | Publication date |
|---|---|---|---|---|---|
| EP 0236124 | A2 | 09-09-1987 | CA | 1335579 C | 16-05-1995 |
| | | | EP | 0236124 A2 | 09-09-1987 |
| | | | JP | H0692441 B2 | 16-11-1994 |
| | | | JP | S62201901 A | 05-09-1987 |
| | | | US | 4965347 A | 23-10-1990 |

EPO FORM P0459

For more details about this annex : see Official Journal of the European Patent Office, No. 12/82

**EP 3 800 206 A1**

**Patent documents cited in the description**

- EP 2018165787 A